# FASCICULE DE BREVET EUROPEEN

(11) **EP 1 575 568 B1**
(45) Date de publication et mention de la délivrance du brevet: **11.04.2018**
(21) Numéro de dépôt: 03814478.8
(22) Date de dépôt: 16.12.2003
(51) Int. Cl.: A61K 9/48

(54) **UTILISATION D AGENTS EPAISSISSANTS POUR LA FABRICATION DE CA PSULES MOLLES, ET PROCEDE DE FABRICATION DE FILMS**
VERWENDUNG VON VERDICKUNGSMITTEL ZUR HERSTELLUNG VON WEICHKAPSELN UND VERFAHREN ZUR HERSTELLUNG VON FILMEN
USE OF THICKENING AGENTS FOR PRODUCING SOFT CAPSULES AND FILM PRODUCTION METHOD

(30) Priorité: 16.12.2002 FR 0215905
(43) Date de publication de la demande: 21.09.2005
(60) Demande divisionnaire: 17173154.0
(73) Titulaire: Paris, Laurence, 03600 Commentry (FR)
(72) Inventeur: Paris, Laurence, 03600 Commentry (FR)
(74) Mandataire: Herrero Herranz, Eva
(86) Numéro de dépôt international: PCT/FR2003/003740
(87) Numéro de publication internationale: WO 2004/060356

(56) Documents cités:
- WO-A-01/07507
- FR-A- 2 767 070
- GB-A- 2 067 214
- US-A1- 2002 019 447
- "GELATIN-FREE SYSTEM FOR SOFT/HARD CAPSULES CONTAINING GELLAN GUM" RESEARCH DISCLOSURE, KENNETH MASON PUBLICATIONS, HAMPSHIRE, GB, no. 332, 1 décembre 1991 (1991-12-01), page 908 XP000274618 ISSN: 0374-4353

## Description

### DOMAINE D'APPLICATION DE L'INVENTION

La présente invention a trait au domaine pharmaceutique, cosmétique et diététique et concerne plus particulièrement la réalisation de films pour l'obtention de capsules molles à partir de substances présentant des propriétés épaississantes. L'invention concerne également un procédé d'obtention de tels films et des dites capsules molles.

### DESCRIPTION DE L'ART ANTERIEUR

Actuellement, la tunique des capsules molles est dans la majorité des cas à base de gélatine soit utilisée pure (gélules) ou en association avec différentes substances (glycérine, sorbitol, etc...). Or, en raison des problèmes potentiels que peut présenter l'origine de la gélatine, en majorité issue d'os de bovins, la maladie dite de la "vache folle" ou BSE ("Bovin Spongiform Encephalite"), fait que l'intérêt de pouvoir substituer un tel produit devient crucial.

De nombreuses substances ont été étudiées dans le but de substituer la gélatine des capsules molles. Dans la majorité des cas, ces substances sont définies comme étant des agents gélifiants donnant naissance à des solutions colloïdales. On entend par " gélifiant " des substances qui après solubilisation à chaud donnent naissance après refroidissement à une masse solide, de structure plus ou moins élastique identique à celle de la gélatine. Cependant ces substances, hormis le fait qu'elles donnent naissance à une masse gélatineuse par refroidissement, doivent répondre à trois critères indispensables à la réalisation des capsules molles, à savoir :
* Etre filmogène, c'est-à-dire que les dites substances doivent former par refroidissement, des films continus par coulage, présentant une épaisseur allant de 0.1 mm à 10 mm et plus;
* Donner naissance à une structure thermoréversible, c'est-à-dire que sous l'effet de la chaleur, ces dites substances redeviennent instantanément liquides et inversement en présence du froid se gélifient très rapidement. Ce paramètre est primordial pour obtenir une soudure parfaite entre deux films soumis à l'action d'une pression et de la chaleur;
* Donner naissance à une structure solide élastique, c'est-à-dire que sous l'effet d'une force, telle qu'une pression ou un étirement, les dites substances gélifiées se déforment et reprennent leur forme initiale après cessation de la force appliquée.

Parmi toutes les substances présentant ces caractéristiques tels que les alginates, certaines gommes comme les gommes Xanthane, l'Agar-agar, les carraghénanes Iota, les carraghénanes Kappa, etc..., très peu ont donné des résultats satisfaisants en raison des paramètres requis au niveau du produit fini entre autre, en ce qui concerne le temps de désagrégation de la capsule molle. Selon les normes fixées par les différentes Pharmacopées, une capsule molle doit présenter un temps de désagrégation de l'ordre de 30 minutes maximum. Dans le cas des capsules molles en gélatine, celui-ci est de l'ordre de 5 à 7 minutes à 37°C dans l'eau. Or, dans la majorité des cas, les substituts de gélatine donnent des valeurs nettement supérieures à 15 minutes et dépassent même les 30 minutes. Cependant, un certain nombre de brevets ont été pris dans le domaine de la capsule molle avec ces dites substances soit utilisées seules comme agent gélifiant ou en association avec un deuxième agent gélifiant ou voire un agent épaississant.

L'une des catégories de substances la plus brevetée est celle des carraghénanes et en particulier les carraghénanes iota et kappa qui présentent tous deux des propriétés gélifiantes. Seul les carraghénanes iota donnent une structure élastique comme la gélatine. Les carraghénanes kappa, hormis le fait qu'ils donnent naissance à une masse solide cassante, non élastique, présentent un autre inconvénient, celui d'un relarguage important d'eau à partir des films formés. Ceci entraîne une rétraction importante des films au cours du temps donc une mauvaise stabilité des produits finis. Ces substances ont donné naissance à un certain nombre de brevets dans le domaine de la capsule molle tels que :
* les brevets JP09025228, JP62289530 et US5342626 dans lesquels les carraghénanes sont en association avec un autre agent gélifiant et leur concentration dans le milieu ne dépasse jamais 5%. De plus, le brevet US5342626 et le brevet JP60012943 font mention de l'utilisation des carraghénanes kappa.
* Le brevet PCT00/10530 (Banner) fait mention de l'utilisation des carraghénanes kappa en tant que seul agent gélifiant mais à une concentration dans le milieu supérieur à 5%
* Le brevet FR2767070 et US6331205 font mention de l'utilisation des carraghénanes iota, aussi utilisés à des concentrations supérieures à 5%.

De tous ces brevets, seuls les brevets FR2767070 et US6331205 ont conduit à une concrétisation industrielle. En effet, les carraghénanes iota à forte concentration se comportent comme la gélatine. Seuls varient les paramètres de température pour travailler la masse. Le temps de désagrégation se situe entre 10 et 15 minutes à 37°C dans l'eau en fonction des ingrédients incorporés dans le film. Ces valeurs sont tout à fait acceptables dans le domaine pharmaceutique.

L'utilisation de ces substances présente un inconvénient lors du procédé de fabrication. En effet, afin d'éviter la prise en masse de la préparation, cette dernière doit être maintenue à une température supérieure à son point de gélification. Dans le cas des carraghénanes, la température doit être maintenue entre 90°C et 100°C. Pour la gélatine, celle-ci est maintenue entre 50°C et 70°C.

Une deuxième catégorie de substances a été testée mais plus particulièrement dans le domaine de la capsule dure. Ce sont les substances dites épaississantes donnant naissance à des solutions pseudo-colloïdales. Par " épaississantes ", il faut entendre des substances qui, à faible ou forte concentration, à chaud ou à froid, augmentent la viscosité du milieu dans lequel elles sont dissoutes sans pour autant donner naissance à une structure gélatineuse à froid ou après refroidissement comme dans le cas de la gélatine. Par conséquent, dans le domaine des capsules molles, ces dites substances ne peuvent a priori aucunement être utilisées car il n'y a pas formation d'une structure solide à froid. Ces substances sont certes considérées dans la majorité des cas comme des agents filmogènes. Mais dans le cas présent, les films sont obtenus par évaporation de l'eau ou du solvant et ils présentent une épaisseur exprimée en microns. En dehors de leur propriété épaississante, ces substances sont aussi généralement utilisées dans les films d'enrobage des comprimés. Ces substances sont nombreuses et appartiennent à la classe :
* des amidons et de leur dérivés,
* des celluloses et de leur dérivés,
* des gommes comme la gomme guar, la gomme de caroube, la gomme arabique, etc.....

Elles sont aussi généralement utilisées comme substituts de la gélatine dans le cas de la fabrication des gélules. C'est le cas de l'hydroxypropylméthylcellulose que l'on retrouve dans toutes les gélules dites d'origine végétale, et qui a donné naissance à de nombreux brevets tels que les brevets US5756123, US4026986, US5431917 et EP0592130.

Le brevet PCT00/18835 (Warner Lambert) propose l'hydroxypropyl amidon pour la fabrication des gélules et à un degré moindre pour les capsules molles. Mais cette dernière est utilisée en association avec une très faible proportion d'un agent gélifiant d'origine végétale, les carraghénanes kappa. L'association des ces deux substances permet d'obtenir une gélification instantanée de la masse d'hydroxypropyl amidon par refroidissement en sortie de bain, reproduisant ainsi le mécanisme de la gélatine.

Dans le domaine de la capsule molle, seuls les brevets de "Swiss caps" PCT01/37817 et EP1103254 décrivent leur obtention à partir de ces dites substances, l'amidon. Cependant l'amidon subit un traitement thermique en présence d'adjuvants conduisant à la formation d'amidons dit thermoplastiques. Ce traitement thermique est obtenu par "cracking" à savoir que les grains d'amidon sous une forte pression et une forte chaleur éclatent et se combinent avec les adjuvants présents dans le milieu. Un tel procédé fait appel à des mélangeurs spéciaux appelés extrudeurs. Des mélangeurs classiques ne peuvent en aucun cas permettre une telle réaction chimique.

### DESCRIPTION DE L'INVENTION

Hormis le brevet de "Swiss Caps" faisant appel à un agent épaississant, l'étude approfondie de tous les autres brevets concernant les capsules molles sans gélatine n'a pas permis de mettre en évidence l'utilisation d'un agent épaississant comme seul composant " gélifié " de la tunique des capsules molles, objet de la présente invention.

A cet effet, les compositions liquides visqueuses, aqueuses ou hydro-alcooliques, tamponnées ou non destinées à la réalisation de films pour la fabrication de capsules molles selon l'invention sont remarquables en ce que leur gélification est obtenue extemporanément à partir d'agents épaississants présentant la propriété unique de se gélifier instantanément au contact de solutions complexantes, l'élasticité des films étant obtenue par l'introduction ou non d'un agent plastifiant, son délitement contrôlé par l'incorporation ou non d'un tensioactif ou d'un polysaccharide, sa conservation assurée ou non par l'addition de conservateurs, lui permettant ainsi de contenir des solutions huileuses et/ou aqueuses.

La présente invention a pour objet de substituer la gélatine des capsules molles par des matériaux appartenant à la catégorie des substances dites épaississantes et de développer le procédé y afférent. Selon une caractéristique particulièrement avantageuse de l'invention, les paramètres requis de structure solide, élastique, filmogène et thermoréversible sont obtenus extemporanément lors du coulage du film par action de substances provoquant une gélification instantanée du dit film, contrairement aux brevets connus où cette caractéristique est recherchée dès la fabrication de la masse d'encapsulation.

Les films ainsi obtenus à froid présentent des caractéristiques similaires au film de gélatine en particulier en ce qui concerne la thermoréversibilité, l'élasticité et le temps de désagrégation par addition ou non d'ingrédients favorisant ces paramètres. Aucun gélifiant de type gélatine, carraghénanes Iota ou Kappa, gomme xanthane, etc... n'est utilisé en association avec ces dites substances épaississantes pour obtenir les propriétés requises décrites précédemment.

L'objet de la présente invention est ainsi basé sur le fait que certaines substances d'origine naturelle ont la propriété de se gélifier instantanément au contact de certaines solutions aqueuses et/ou hydro-alcooliques plus ou moins concentrées en ions. On entend par " ions " des éléments ionisés tels que :
* les ions hydrogènes (H⁺) définissant les acides,
* les ions hydroxydes (OH⁻) définissant les bases
* les anioniques et/ou les cationiques, définissant les sels (calcium, sodium, phosphates, etc...)

Ces solutions sont définies comme étant les " agents gélifiants" et plus particulièrement pour se distinguer dans le contexte de la présente invention, comme "agents complexants "

Les structures ainsi obtenues sont :
* des films thermoréversibles permettant ainsi la soudure de deux unités sous l'effet d'une pression et de la température, lors de l'encapsulation de principes actifs;
* des films plus ou moins élastiques en fonction de l'agent gélifiant ou complexant mis en oeuvre et de la composition de desdits films, permettant ainsi un remplissage par injection d'une solution de principe actif à encapsuler.

Le procédé, également objet de la présente invention, utilise l'avantage que ces dites substances d'origine naturelle permettent de travailler à une température très inférieure à leur point de fusion et à des concentrations en matières solides nettement plus importantes que les gélifiants classiquement utilisés tels que la gélatine, les carraghénanes (Iota et Kappa), et les compositions gélifiantes décrites dans les différents brevets étudiés. Ainsi, l'énergie consommée lors de l'encapsulation de principes actifs est pratiquement réduite à néant en raison de l'absence de la nécessité de maintenir la chaleur de la préparation filmogène pour éviter sa gélification in situ, comme souvent observé dans le cas de la gélatine, des carraghénanes et des autres compositions gélifiantes.

L'obtention des dits films faisant l'objet de la présente invention fait appel à des substances épaississantes d'origine végétale ou issues du métabolisme de micro-organismes et très utilisées dans le domaine agro-alimentaire et pharmaceutique, qui au contact de certaines solutions aqueuses et/ou hydro-alcooliques, se gélifient instantanément.

Ces agents épaississants sont généralement très solubles à froid et appartiennent à la classe des polysaccharides contenant sur leur chaîne polymérique au moins une molécule de glucose, de galactose, de mannose et/ou d'acide glucuronique ou un mélange des trois et/ou des dérivés des trois.

Ces substances à pouvoir gélifiant induit peuvent être utilisées seules ou en association avec d'autres excipients non gélifiants ayant pour but
* d'améliorer l'élasticité du film,
* de renforcer la soudure de deux films,
* d'agir sur le temps de désagrégation de la tunique,
* d'assurer la propreté microbiologique,
* d'améliorer l'aspect final de la capsule.

De ce fait, en fonction de la composition du film, la libération d'un principe actif inclus dans une telle capsule peut varier entre 5 minutes à 24 heures.

Les agents épaississants d'origine végétale sont nombreux mais ne donnent pas tous des gels au contact d'agents complexants, tel que défini précédemment ou les gels formés ne présentent pas les propriétés requises pour réaliser des capsules molles.

Certains présentent ces propriétés lorsqu'ils sont associés avec d'autres substances ayant des propriétés gélifiantes. C'est le cas de la gomme de caroube, agent épaississant, qui en présence des carraghénanes Kappa, agent gélifiant, va donner naissance à un gel élastique. Dans ce cas la gomme de caroube joue le rôle de plastifiant au sein du gel de carraghénanes Kappa qui présente en temps normal une structure cassante.

Dans d'autres cas, il peut y avoir formation d'un gel au contact "d'agents gélifiants" mais ce dernier n'est pas thermoréversible. Tel est le cas des alginates qui en présence d'ions calcium et en milieu acide vont donner naissance à des gels mais sous l'effet de la chaleur, ces derniers ne vont pas redevenir liquides comme dans le cas de la gélatine ou des carraghénanes. La même observation est faite avec les pectines en milieu acide. Il y a formation d'un gel mais non thermoréversible.

Les épaississants d'origine végétale sont extraits des plantes, des fruits et de certaines algues. Ces produits sont très largement utilisés dans l'industrie alimentaire, pharmaceutique et cosmétique. Leur rôle principal est de maintenir en suspension des particules de par l'épaississement de la phase liquide.

En plus d'augmenter la viscosité du milieu, ces dites substances ont la capacité à se prendre instantanément en masse lorsqu'elles sont mises en contact avec des "agents gélifiants".

Ce sont :
- la gomme arabique et leur dérivés
- les carraghénanes Lambda.
   * La gomme arabique est une exsudation de la sève à partir des branches et du tronc de différentes espèces d'acacias, *Vera, Nilotica, Senegaensis, Verek, Seyal, etc....* Elle se présente en larmes blanches, en gros morceaux de couleur rouge, ou en morceaux irréguliers, anguleux, brisés, brillants de couleur blanc rougeâtre. La gomme arabique est soluble dans l'eau. Une solution aqueuse à 5% de gomme arabique provenant d'acacia Verek est dextrogyre. La concentration à saturation à 25°C est de 37g pour 100g d'eau. La gomme arabique est constituée en majeure partie voire en totalité par du (-)-arabinose, du (+)-galactose, du (-)-rhamnose et de l'acide (+)-glycuronique. La gomme arabique existe sous forme de nébulisât dans le but de faciliter sa solubilisation. Elle est utilisée dans le domaine alimentaire et pharmaceutique comme agent de suspension, d'émulsifiant et de liant dans la fabrication des comprimés. Au contact de l'alcool, du borate de sodium, et du silicate de sodium, la gomme arabique donne naissance instantanément à un gel.
   * Les carraghénanes sont connus depuis plus de 600 ans dans le domaine médical et en alimentation en particulier pour leur propriété originale qui consistait à gélifier le lait par simple chauffage. Ce sont des polysaccharides, polymères du galactose plus ou moins sulfatés. Les carraghénanes sont extraits à partir de différentes algues : Chondrus crispus, Gigartina stellata, Gigartina acicularis, Gigartina skottsbergii, Gigartina pistillata, Gigartina chamissoi, Iridea, Eucheuma cottoni, Eucheuma spinosum. Le procédé d'extraction mis en oeuvre conduit à différents types de carraghénanes dont le squelette de base est une chaîne de D-galactoses liés alternativement en α-(1-3) et α-(1-4) . Les différentes qualités sont dues à la quantité et à la position des groupements sulfates et à la présence ou non d'un pont 3,6 anhydro sur le galactose lié en 1 et 4. La proportion des différents groupes sulfates et du pont 3,6 anhydrogalactose a permis d'isoler différents types de carraghénanes. Ce sont les carraghénanes Iota, Kappa, Lambda, Bêta, Nu et Mu.

Les formes Lambda présentent beaucoup de groupements soufrés comparées aux formes Kappa. Les formes Iota sont intermédiaires.

Les formes Bêta, Mu et Nu sont en plus faibles quantités et sont considérées comme impuretés diminuant l'effet gélifiant des formes Iota et Kappa.

Le type de carraghénanes retenu pour la présente invention est le Lambda.

Comparés aux carraghénanes Iota et Kappa, les carraghénanes Lambda ne présentent aucun phénomène de synérèse. Elle ne présente pas de propriétés gélifiantes mais épaississantes. Par contre au contact d'une solution de sels alcalins et/ou alcalino-terreux, ces carraghénanes donnent naissance à une masse gélifiée. Cette réaction est instantanée et se produit sans traitement thermique préalable.

Les épaississants d'origine cellulaire sont extraits des milieux de cultures dans lesquels ont été cultivés des micro-organismes spécifiques. En fonction de la composition du milieu, ces micro-organismes vont sécréter différents constituants en plus ou moins en grandes quantités. Les gommes issues de ces milieux sont :
- les gommes Pullulan et leurs dérivés
- les gommes Rhamsan et leurs dérivés
- et les gommes Wellan et leurs dérivés.

Comme les carraghénanes, ces différentes gommes, en fonction de groupements fixés sur le squelette de base, ont des propriétés gélifiantes ou épaississantes.
* Les gommes Rhamsan et Wellan sont obtenues à partir d'un micro-organisme *"Alcaligenes species".* Les gommes Rhamsan sont des hétéropolysaccharides composés d'unités tétrasaccharidiques. L'unité de base est constituée de 3 molécules de D-glucose liées entre elles en β(1 → 4) configuration, la dernière étant liée en α (1→4) configuration avec une molécule de L-rhamose. Que ce soient les gommes Rhamsan ou Wellan, leur utilisation comme agent épaississant est très variée. En effet, elles peuvent être utilisées dans les produits pharmaceutiques comme dans les produits alimentaires ou encore dans le domaine du bâtiment.
* Les gommes Pullulan sont sécrétées par *"Aureosbasidium pullulans".* Ce sont des polymères linéaires dont le squelette principal est constitué d'un enchaînement de molécules de maltotriose liées entre elles en une configuration α(1→6). Le maltotriose est constitué de trois molécules de glucose. En raison des différents groupements hydroxyles présents sur le polymère, de nombreux dérivés ont pu être synthétisés par ce micro-organisme en fonction de la nature de son milieu nutritionnel. Comme précédemment, ces gommes sont largement utilisées dans différents domaines allant de la pharmacie à la fabrication des peintures. Ce sont de très bons agents filmogènes présentant une très bonne élasticité.

Que ce soient les épaississants d'origine végétale ou issu du métabolisme de certains micro-organismes, leur proportion à mettre en oeuvre pour obtenir le même résultat qu'avec la gélatine peut varier d'une concentration de 2% à 80% en masse par rapport à la masse totale de la préparation.

Chacune de ces dites substances peut être utilisée comme seul " gélifiant extemporané " ou en association avec une ou plusieurs autres, tels que :
- carraghénanes Lambda/gomme arabique pour renforcer la soudure lors de la fabrication des capsules molles en raison des propriétés adhésives de la gomme arabique,
- carraghénanes Lambda/gomme Pullulan pour moduler la libération du principe actif encapsulé,
- etc....

La proportion des autres " gélifiants extemporanés " peut varier de 10% à 90% en masse par rapport à la masse totale d'agents épaississants.

Suivant l'agent épaississant mis en oeuvre, le milieu de dissolution peut être aqueux ou hydro-alcoolique. La proportion de la phase alcoolique peut varier de 10% à 90% en masse par rapport à la masse totale de la préparation.

En fonction de l'agent épaississant utilisé, l'addition de certains ions permet une meilleure hydratation du polymère saccharidique, tel est le cas des carraghénanes Lambda.

Les agents favorisant la solubilisation des carraghénanes appartiennent à la classe des ions alcalins et des ions alcalino-terreux (sodium et potassium). Ce sont en autres :
- les sels sodiques et potassiques des acides chlorhydrique, sulfurique, nitrique, phosphorique, citrique et dérivés;
- et les hydroxydes de sodium et de potassium.

La proportion d'ions alcalins et alcalino-terreux pouvant être introduite dans le milieu varie entre 0% à 50% en masse par rapport à la masse totale de la préparation.

La phase aqueuse, en fonction de l'agent épaississant utilisé peut être tamponnée. On entend par " tamponnée ", une phase aqueuse qui est capable d'absorber des variations de pH acide ou basique en fonction de leur composition, de manière à maintenir le pH du milieu constant. Ceci est d'autant plus important que certains agents épaississants se dégradent facilement en fonction du pH final du milieu, accentué par une exposition prolongée à la chaleur. Tel est le cas des carraghénanes Lambda qui, en présence de dextrose en milieu neutre, subit une hydrolyse progressive dans le temps, accrue par l'action de la chaleur. En effet en milieu neutre et sur une période de vingt quatre heures, on observe une diminution de la viscosité du milieu par une hydrolyse progressive des carraghénanes libérant dans le milieu des radicaux acides. Dans le cadre de solutions tampons acide, les compositions suivantes peuvent être les couples:
- tampon acide chlorhydrique/chlorure de sodium ou acide chlorhydrique/phtalate de potassium ou acide chlorhydrique/glycocolle.
- tampon acide citrique/citrate ou acide citrique/hydroxyde de sodium
- tampon acide lactique/lactate.

La proportion des différents composants permet de maintenir un pH acide compris entre 2 et 5.

Dans le cas des carraghénanes Lambda, la meilleure stabilité est observée en milieu neutre ou basique. Les solutions tampons qui peuvent être ainsi utilisées répondent aux compositions suivantes :
- tampons phosphates : phosphate de sodium ou de potassium
   * tampon carbonates : bicarbonate/carbonate
   * tampon phtalate : diphtalate de potassium/acide chlorhydrique

La valeur, du pH du milieu tamponné peut varier de 5 à 12.

Comme il a été mentionné précédemment, les films entrant dans la composition des capsules molles doivent présenter une certaine élasticité pour permettre une injection de la solution à encapsuler sous pression. Ces substances sont d'autant plus importantes que les agents épaississants retenus pour la réalisation de capsules molles sans gélatine, ne possèdent pas la propriété intrinsèque de former des films élastiques. L'élasticité des films est obtenue par l'utilisation d'agents plastifiants qui appartiennent à la classe des polyols : glycérol, sorbitol, maltodextrines, dextrose, manitol, xylitol, lactitol, propylène glycol, polyoxyéthylène glycol 400 à 6000, glycérides naturels et hemi-synthétiques et leur dérivés. La quantité de ces substances introduites dans la préparation est telle que le coefficient d'élasticité du film peut varier de 1 à 5 (1 à 5 fois la longueur initiale) . La proportion de ces substances plastifiantes pouvant être introduite dans le milieu varie entre 0% et 50% en masse par rapport à la masse totale de la préparation.

Tel qu'il a été défini initialement, les capsules molles doivent répondre à certaines caractéristiques en qualité de temps de désagrégation. Afin d'atteindre les objectifs en la matière, un certain nombre d'excipients peuvent être introduits dans le milieu pour contrôler ce paramètre. Deux catégories de produits peuvent être retenues :
- les tensioactifs,
- les agents désintégrants.

Les tensioactifs facilitent la mouillabilité des produits avec lesquels ils sont en contact. Ceux utilisés dans la présente invention appartiennent à différentes classes de produits :
- les non ioniques. Ce sont :
   * des esters de sorbitane : polysorbates, spans, tweens, etc...
   * des acides gras polyéthoxylés : stéarate de PEG 8 au stéarate de PEG 100;
   * des alcools gras polyéthoxylés : mélange d'éther de monolaurate de PEG ayant de 4 à 23 groupes oxyéthylènes sur la chaîne polyoxyéthylénique, etc...
   * des esters de glycol : stéarate de méthylglycol;
   * des esters de glycérol : monostéarate de glycérol, etc...
   * des esters de PEG;
   * des esters de saccharose;
   * des éthers d'alcool gras et de PEG : Brij;
   * des éthers d'alkyl phénol et de PEG;
   * des tensioactifs présentant une fonction amide :
      ∘ monoéthanolamide d'acide gras de coprah, d'acide laurique, etc...
      ∘ diéthanolamide d'acide myristique, d'acide laurique, etc...
      ∘ mono-isopropanolamine d'acide laurique.
- les ioniques. Ce sont :
   * des dérivés sulfatés : le laurylsulfate de sodium et ses dérivés;
   * des dérivés sulfonés : dodécylsulfosuccinate de sodium et ses dérivés;
   * des ammoniums quaternaires : chlorure de cétyltriméthylammonium, laurylpyridinium, distéaryldiméthylammonium, etc...
- les amphotères sont : bétaïne d'ammonium d'alkyldiméthyle de coprah, dérivés d'amides d'acide gras à structure bétaïnique, acide lauryl-á-iminodipropionique et ses dérivés, acide lauryl-myristyl-α-aminopropionique et ses dérivés, etc...

La quantité de ces substances introduites dans la solution des agents épaississants est telle que le temps de désagrégation peut varier de 3 minutes à 8 heures. Ces quantités peuvent varier de 0 à 20% en masse par rapport à la masse totale de la préparation. Cependant dans certains cas, la seule présence de tensioactifs ne suffit pas. Des agents de désintégration permettent d'améliorer le temps de désagrégation. De nombreuses substances peuvent jouer ce rôle. Parmi celles-ci les amidons ont été retenus en particulier leur dérivés car ils présentent de bonnes solubilité dans les milieux de solubilisation des substances épaississantes. Ainsi les amidons natifs dits solubles sont retenus comme agent désintégrant dans la dite invention ainsi que leurs produits dérivés issus de :
- modifications physiques : pré-gélatinisation
- modifications chimiques :
   * réaction de dextrénisation chimiques ou enzymatiques
   * hydrolyse acide
   * réaction d'oxydation
   * réaction de substitution par :
      ∘ l'acide phosphorique
      ∘ l'acide adipique
      ∘ l'acide acétique
      ∘ des groupements hydroxypropyl ou hydroxyéthyl.

Ces différents agents de désintégration peuvent être obtenus à partir des amidons de blé, de riz, de maïs, de manioc de pomme de terre. Les quantités utilisées peuvent varier de 0 à 50% en masse par rapport à la masse totale de la préparation. Des adjuvants de conservation, des colorants et opacifiants peuvent aussi être introduits dans la composition. La proportion de conservateurs peut varier de 0.01 à 10% en masse par rapport à la masse totale de la préparation. Les colorants peuvent être hydrosolubles ou fixés sur laque d'alumine ou autre support. Le taux optimum requis se situe entre 0.01% et 5% en masse pour les colorants et de 1 à 10% en masse pour les opacifiants, par rapport à la masse totale de la préparation.

Les solutions ainsi réalisées présentent une viscosité à froid comprise entre 200 millipascales et 1.000.000 millipascales et une concentration en matières solides pouvant aller de 10% à 80% en masse par rapport à la masse finale de la composition.

A partir de ces solutions, des films peuvent être réalisés présentant une épaisseur au moment de la coulée de l'ordre de 0.5 mm à 4.0 mm d'épaisseur. Leur gélification induite par des agents complexants fait que les films ainsi obtenus présentent des caractéristiques physiques similaires à ceux en gélatine ou en carraghénanes Iota, tant sur le plan de l'élasticité, de leur épaisseur, du temps de désagrégation, de leur soudure, que sur le plan aspect.

Les " agents gélifiants " ou complexants induisant la gélification extemporanée des préparations précédentes appartiennent à deux classes de produits en fonction des substances épaississantes mises en oeuvre :
- les alcools tel que l'éthanol, le méthanol, le propanol, l'isopropanol, le butanol, dans le cas de la gomme arabique
- les sels de :
   * calcium
   * baryum
   * titane
   * zinc
   * aluminium
   * soufre
   * silice
d'acides minéraux et/ou organiques, ainsi que les oxydes, les hydroxydes et les carbonates correspondant, dans le cas des carraghénanes Lambda et des gommes pullulan, rhamsan et wellan.

Dans le cas des alcools, ceux-ci peuvent être utilisés purs ou dilués dont la concentration peut varier de 10% à 90% en masse par rapport au volume final de la solution d'agent hydro-alcoolique complexant.

Dans le cas des sels et des oxydes agissant en tant qu'agents complexants, ils sont préalablement dissous :
- en milieu aqueux, acide, neutre ou basique,
- ou en milieu hydro-alcoolique pour renforcer leur action.

Leur concentration peut varier de 1% jusqu'à utilisation de solutions dite saturées.

Que ce soient les acides ou les hydroxydes utilisés pour solubiliser les sels et les oxydes, leur normalité ou concentration en milieu aqueux varie de 0.01N à 10N.

Les acides retenus pour la présente invention sont :
- d'origine minérale : acide chlorhydrique, sulfurique, nitrique, phosphorique, etc....
- d'origine organique : acide acétique, formique, lactique, citrique, sucçinique, etc.....

La solidité de la soudure de deux films ainsi réalisés sera fonction du degré de leur gélification, par conséquent du temps de contact entre le film et l'agent complexant. En fonction de la nature de l'agent épaississant et de l'agent complexant mis en oeuvre, le temps de contact peut varier entre 10 secondes et 10 minutes.

La présente invention porte également sur le procédé de fabrication de la masse, des films et des capsules molles. Contrairement aux procédés actuellement mis en oeuvre pour la réalisation de capsules molles qu'elles soient en gélatine, carraghénanes (Kappa ou Iota) ou en amidon thermoplastique, la consommation d'énergie est réduite. En effet, en raison de la gélification intrinsèque des produits cités ci-dessus, il est nécessaire de travailler à chaud pour éviter la prise en masse de la gélatine et des carraghénanes dans les canalisations. Dans le cas des amidons thermoplastiques, les extrudeurs mis en oeuvre consomment beaucoup d'énergie afin de réaliser in situ la transformation chimique de l'amidon natif.

A contrario, le procédé de fabrication, faisant l'objet de la présente invention, est original en ce que les agents épaississants présentent des propriétés gélifiantes extemporanées ou induites, évitant ainsi la prise en masse de la préparation dans les canalisations. La préparation de la masse se fait dans la majorité des cas à froid. Un chauffage peut être nécessaire pour accélérer l'hydratation ou la solubilisation des agents épaississants et de certains autres additifs. Les températures mises en oeuvre pour favoriser l'hydratation ou la solubilisation ne dépassent jamais 90°C. Une fois cette étape réalisée, il n'est plus nécessaire de poursuivre le chauffage. Les mélangeurs utilisés pour la réalisation de cette masse sont classiques et du type mélangeur malaxeur.

Une fois la masse réalisée, elle subit une étape classique de dégazage sous vide pour éliminer l'air susceptible de former des bulles lors de la réalisation des films. Cette étape de dégazage peut être simultanée à la fabrication de la masse en travaillant sous vide. La masse dégazée est transférée vers les systèmes de formation des films soit par gravité simple soit par pression en utilisant soit des vis sans fin ou une presse.

Dès la formation du film, celui-ci est mis en contact avec la solution complexante.
Ce contact peut se faire par :
- trempage
- pulvérisation
- ou par pulvérisation/trempage.

Selon le matériel retenu pour fabriquer les films, le trempage et/ou la pulvérisation peuvent se faire simultanément sur les deux faces du film ou alternativement par décollement spontané du film gélifié. En effet, il a été observé, dans le cas des carraghénanes Lambda, que les films gélifiés formés se décollent instantanément de leur support par pénétration par capillarité de la solution complexante entre le film et le support permettant ainsi la gélification de l'autre face.

Les exemples de système de fabrication des films figurant ci-après sont des schémas possibles de procédés pour répondre au mode de fabrication défini et ils ne les limitent en aucune façon.

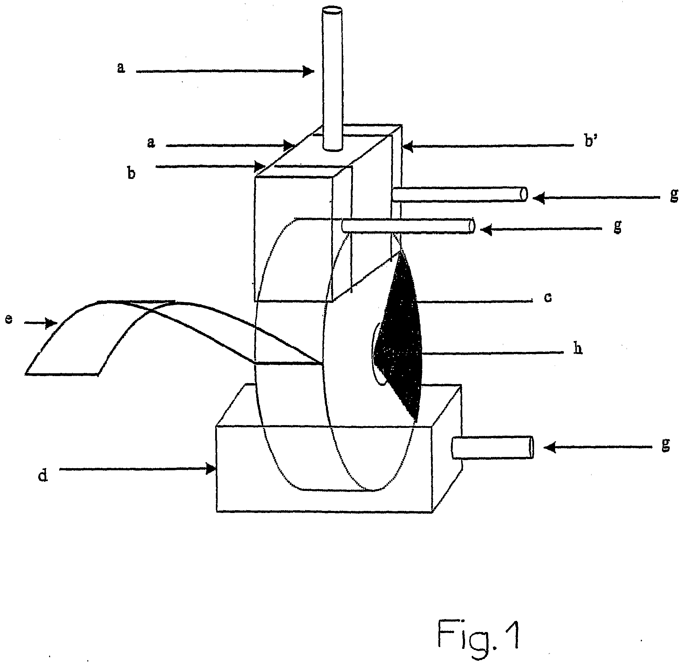

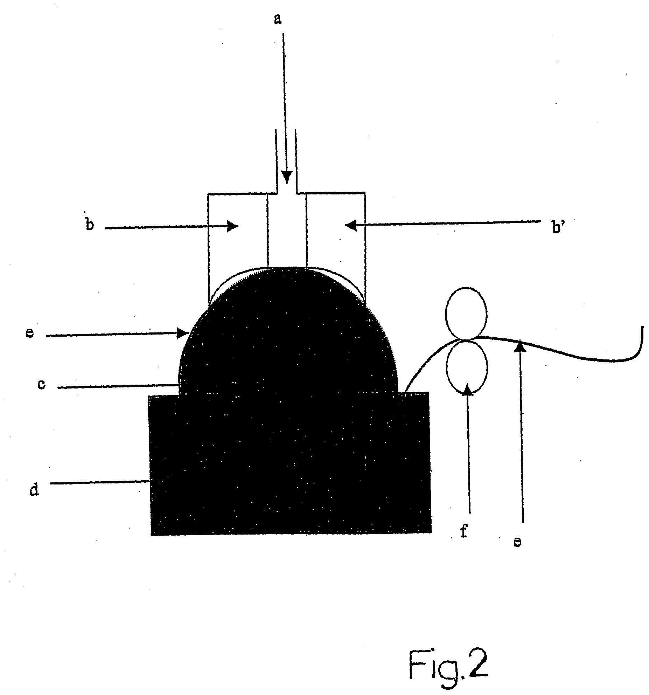

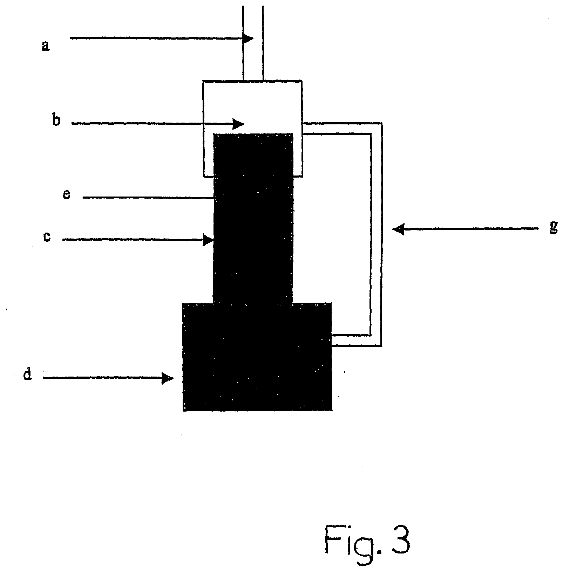

Les figures 1 à 3 par des vues en perspective, en coupe de face et en coupe transversale respectivement, illustrent le " système tambour " classiquement utilisé pour la fabrication des capsules molles. Il a été adapté deux injecteurs (b et b') en position haute pour la pulvérisation de la solution complexante et un bain de trempage (d) pour finaliser la gélification du film après décollement du tambour.

Dans le cas présent la gélification du film se fait d'abord sur une face puis sur l'autre.

Ainsi à partir du système d'alimentation (a), la masse fabriquée donne naissance à un film (e) qui est entraîné par un tambour (c). A partir de la buse (b), le film (e) se gélifie face extérieure. Simultanément, la solution complexante à partir de la buse (b') entraîne par ruissellement le long du tambour et par capillarité, la gélification de la face interne du film (e) et son décollement du tambour (c) . En fonction de la hauteur du système d'entraînement (référencée f et reportée uniquement sur le dessin de la figure 1) du film gélifié, ce dernier peut séjourner plus au moins longtemps dans le bac de trempage (d) en fonction des critères recherchés.

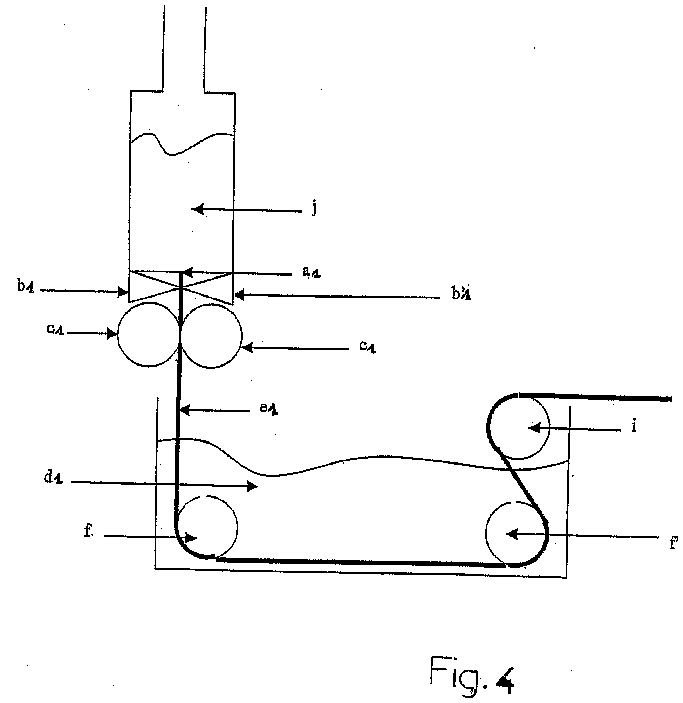

Le système représenté en coupe sur le dessin de la figure 4 permet une gélification instantanée des deux faces en sortie de la cuve de fabrication de la masse. C'est ainsi qu'en sortie (a1), la masse fabriquée donne naissance à un film (e1) instantanément gélifié des deux côtés, par pulvérisation de la solution complexante à partir des deux gicleurs (b1) et (b1') disposés de part et d'autre de l'orifice de sortie (a&). Un système d'entraînement (c1, f, f' et i) du film permet ou non de le faire séjourner dans un bain de trempage (d1) pour parfaire sa gélification. Le film gélifié ainsi formé peut être séché dans un courant d'air dont la température peut varier de -10°C à 70°C, lors de son processus de transfert vers la partie " encapsulation " de la machine, tel que cela est indiqué dans les brevets FR9710190 et US6331205.

Après séchage, les films gélifiés présentent une épaisseur s'échelonnant entre 0.2 et 2.0 mm d'épaisseur. Leur pouvoir de rétraction après séchage se situe entre 0% et 20%.

Les films ainsi obtenus peuvent être lubrifiés pour faciliter leur transfert vers les moules d'encapsulation qu'ils soient rotatifs dans le cas du procédé de RP Scherer ou alternatifs dans le cas du procédé de Norton. Les lubrifiants pouvant être utilisés sont :
- des huiles alimentaires classiques : huile d'arachide, de tournesol, d'olive, etc...
- des émulsifiants tels que les esters de glycérol et polyoxyéthylène glycol, des triglycérides, des esters de propylène glycol et leur dérivés.

Ces lubrifiants peuvent être utilisés purs ou dilués avec une dilution s'échelonnant de 10 à 75%.

Les films ainsi obtenus pour la fabrication des capsules molles peuvent encapsuler des poudres, des solutions et des suspensions dont les véhicules sont constitués par un mélange ou non :
- d'huiles : huile d'arachide, tournesol, d'olive... et de type labrafil;
- de polyoxyéthylèneglycol : PEG 400, 600 etc...
- de propylène glycols;
- d'émulsionnants : polysorbates, lécithine soja;
- d'agents de suspension comme des huiles hydrogénées;
- de solutions aqueuses contenant un ammonium quaternaire.

Lors de l'encapsulation des ces dites substances, la température de fermeture des capsules doit être comprise entre 50°C et 100°C. Le procédé de fermeture des capsules peut mettre en oeuvre des moules chauffants dont la température doit être comprise entre 60°C et 110°C.

Les exemples de réalisation de films figurant ci-après sont uniquement des formules possibles de compositions selon l'invention données à titre non limitatif.

### Exemple n°1

| | |
|---|---|
| Gomme Arabique | 40 g |
| Glycérine | 10 g |
| Eau Q.S | 100 g |

### Exemple n°2

| | |
|---|---|
| Carraghénanes Lambda | 10.0 g |
| Chlorure de sodium | 3.0 g |
| Glycérine | 10.0 g |
| Eau Q.S | 100 g |

Solution de complexation : solution de chlorure de calcium à 50%

### Exemple n°3

| | |
|---|---|
| Carraghénanes Lambda | 10.00 g |
| Chlorure de sodium | 3.00 g |
| Glycérine | 15.00 g |
| Jaune orangé S | 0.05 g |
| Eau Q.S | 100.00g |

Solution de complexation : solution de chlorure de calcium à 50%

### Exemple n°4

| | |
|---|---|
| Carraghénanes Lambda | 10.00 g |
| Sorbitol | 20.00 g |
| Polysorbate 80 | 1.50 g |
| Eau Q.S | 100.00 g |

Solution de complexation : solution de chlorure de calcium à 50%

### Exemple n°5

| | |
|---|---|
| Carraghénanes Lambda | 10.00 g |
| Chlorure de potassium | 2.00 g |
| Glycérine | 15.00 g |
| Polysorbate 80 | 1.50 g |
| Parahydroxybenzoate de méthyle sodé | 0.12 g |
| Parahydroxybenzoate de propyle sodé | 0.03 g |
| Eau Q.S | 100.00 g |

Solution de complexation : solution de chlorure de calcium à 50%

### Exemple n°6

| | |
|---|---|
| Carraghénanes Lambda | 39.500 g |
| Chlorure de sodium | 1.918 g |
| Glycérine | 30.00 g |
| Polysorbate 80 | 6.000 g |
| Parahydroxybenzoate de méthyle sodé | 0.36 g |
| Parahydroxybenzoate de propyle sodé | 0.09 g |
| Phosphate monosodique | 0.390 g |
| Phosphate disodique | 7.320 g |
| Eau Q.S | 300.000 g |

Solution de complexation : solution de chlorure de calcium à 50%

### Exemple n°7

| | |
|---|---|
| Carraghénanes Lambda | 39.500 g |
| Glycérine | 30.00 g |
| Hydroxypropylamidon | 60.000 g |
| Parahydroxybenzoate de méthyle sodé | 0.36 g |
| Parahydroxybenzoate de propyle sodé | 0.09 g |
| Phosphate monosodique | 0.390 g |
| Phosphate disodique | 7.320 g |
| Eau Q.S | 300.000 g |

Solution de complexation : solution de chlorure de calcium à 50%

### Exemple n°8

| | |
|---|---|
| Carraghénanes Lambda | 39,500 g |
| Chlorure de sodium | 1.918 g |
| Sorbitol | 30.00 g |
| Maltodextrine | 40.000 g |
| Parahydroxybenzoate de méthyle sodé | 0.36 g |
| Parahydroxybenzoate de propyle sodé | 0.09 g |
| Phosphate monosodique | 0.390 g |
| Phosphate disodique | 7.320 g |
| Eau Q.S | 300.000 g |

Solution de complexation : solution de chlorure de calcium à 50%

Basé sur l'exemple n° 7, dans une cuve de 500 litres à double paroi en inox, pourvue d'un système d'agitation et du vide, on introduit 163,00 kg d'eau, le phosphate monosodique, le phosphate disodique et les conservateurs et l'agitation est maintenue jusqu'à dissolution complète des ingrédients. La température de la préparation est progressivement amenée à 90°C pour faciliter l'hydratation de l'hydroxypropylamidon lors de son introduction dans le milieu. Après homogénéisation, la glycérine est ajoutée ainsi que les carraghénanes Lambda. La température est maintenue à 90°C pendant 3 à 4 heures pour permettre la parfaite hydratation des carraghénanes Lambda. La masse ainsi fabriquée peut être stockée plus de 48 heures à 25°C.

Après dégazage de la préparation, celle-ci est transférée vers les machines d'encapsulation à une température minimum de 50°C.

Par simple gravité ou par pression, il y a formation, selon le procédé de fabrication du film :
- d'un dépôt de masse sur les tambours dont leur température est de l'ordre de 10 - 15°C,
- ou d'un film épais, non gélifié en sortie de trémie d'alimentation entre deux cylindres dont leur température est de l'ordre de 10 - 15°C.

La gélification du film est ensuite obtenue par pulvérisation de la solution de chlorure de calcium à 50% soit,selon le procédé de fabrication du film :
- à la surface du tambour, côté externe du film puis interne
- ou bien par pulvérisation de chaque côté du film.

Quel que soit le mode de fabrication du film, celui-ci après gélification est transféré directement vers la partie encapsulation après passage ou non dans le bain de trempage durant une période de 30 secondes à 1 minute. Le film obtenu subit un séchage dans un courant d'air dont la température est réglée entre +4°C et 30°C. Les capsules molles sont obtenues par scellage à chaud des films après lubrification soit :
- par le procédé classiquement utilisé, chauffage préalable du film entre 70°C et 100°C avant soudure par pression,
- ou par chauffage des moules à une température comprise entre 70°C et 100°C.

## Revendications

1. Procédé de fabrication des films pour la fabrication de capsules molles permettant de contenir des solutions huileuses et/ou aqueuses à partir de compositions liquides visqueuses, aqueuses ou hydro-alcooliques, tamponnées ou non (A),
**caractérisé en ce qu'**il consiste :
i) à réaliser à froid ou à une température n'excédant pas 90°C, une solution pseudo-colloïdale par dispersion sous vide des agents épaississants, les carraghénanes Lambda, avec une proportion dans le milieu de la composition (A) liquide visqueuse, aqueuse ou hydro-alcoolique, tamponnée ou non, supérieure à 2%, contenant:
a) des ions alcalins ou alcalino-terreux
b) un plastifiant,
c) un tensioactif
et/ou
d) un agent désintégrant,
ii) à maintenir la solution résultant de i) à 25°C pendant son stockage,
iii) à réaliser des films pour les capsules molles avec une température maintenue à au moins 50°C,
iv) à gélifier la masse du film en appliquant une solution complexante (B) par pulvérisation et/ou par trempage, soit simultanément sur les deux faces du film, soit alternativement après décollement du film de son support,
les dites solutions complexantes (B) étant des solutions salines de sels d'acides minéraux ou organiques ainsi que des hydroxydes, des oxydes et des carbonates de calcium, baryum, titane, zinc, aluminium, soufre et de silices;
la gélification des compositions (A) liquides visqueuses, aqueuses ou hydro-alcooliques est obtenue extemporanément à partir des agents épaississants lambda carraghénanes présentant la propriété unique de se gélifier instantanément au contact de la dite solution complexante (B).

2. Procédé de fabrication selon la revendication 1, **caractérisé en ce que** les carraghénanes Lambda sont a une concentration de 2% à 80% en masse par rapport à la masse totale de la composition (A).

3. Procédé de fabrication selon la revendication 1, **caractérisé en ce que** la teneur en tensioactifs varie de 0 à 20% en masse par rapport à la masse totale de la composition (A).

4. Procédé de fabrication selon la revendication 1, **caractérisé en ce que** la proportion d'agent plastifiant varie de 0 à 50% en masse par rapport à la masse totale de la composition (A)

5. Procédé de fabrication selon la revendication 1, **caractérisé en ce que** la concentration en matière solide est comprise entre 10% et 80% en masse par rapport à la masse finale de la composition (A).

6. Procédé de fabrication selon la revendication 1, **caractérisé en ce que** les ions alcalins sont choisis parmi le sodium et le potassium.

7. Procédé de fabrication selon la revendication 1, **caractérisé en ce que** l'agent plastifiant appartient à la classe des polyols du type glycérol, sorbitol, maltodextrines, dextrose, manitol, xylitol, lactitol, propylène glycol, polyoxyéthylène glycol 400 à 6000, glycérides naturels et hemi-synthétiques et leur dérivés.

8. Procédé de fabrication selon la revendication 1, **caractérisé en ce que** le tensio-actif assurant le contrôle du délitement du film appartient aux classes des tensio-actifs ioniques, non ioniques et amphotères.

9. Procédé de fabrication selon la revendication 1, **caractérisé en ce que** les agents désintégrants sont des amidons solubles de pomme de terre, de maïs, de riz, de manioc, de blé ayant subi ou non des transformations chimiques ou physiques.

10. Procédé de fabrication selon la revendication 1, **caractérisé en ce que** le temps de gélification du film au contact de la solution complexante (B) est compris entre 10 secondes à 10 minutes.

11. Procédé de fabrication selon les revendications 1 à 10, **caractérisé en ce que** les films obtenus dans l'étape (iv) à partir desdites compositions visqueuses, aqueuses ou hydro-alcooliques, tamponnées ou non (A), sont lubrifiés par des huiles alimentaires classiques ou des esters de glycérol et polyoxyéthylène glycol, des triglycérides, des esters de propylène glycol et leurs dérivés ou des solutions diluées de ces différents produits.

12. Procédé de fabrication selon les revendications 1 à10 , **caractérisé en ce que** l'encapsulation des principes actifs à partir de ces films se fait par soudure à chaud sous
pression des deux films, sous une température comprise entre 50°C et 100°C.

13. Procédé de fabrication selon les revendications 1 à 10, **caractérisé en ce qu'**il consiste à faire subir au film gélifié, une opération de séchage dans un courant d'air dont la température est comprise entre - 10°C et +70°C.

14. Procédé de fabrication selon les revendications 1 à 10, **caractérisé en ce qu'**il consiste à faire subir à la masse réalisée (étape iii), préalablement à l'étape de gélification (étape iv), une étape de dégazage sous vide pour éliminer l'air susceptible de former des bulles lors de la réalisation des films.

15. Procédé de fabrication selon les revendications 1 à 10, **caractérisé en ce que** dans l'étape iii), la masse est transférée vers les systèmes de formation des films soit par gravité simple soit par pression en utilisant des vis sans fin ou une presse.

## Patentansprüche

1. Verfahren zur Herstellung von Filmen für die Herstellung von Weichkapseln zum Aufnehmen öliger und/oder wässriger Lösungen ausgehend von flüssigen viskosen, wässrigen oder wässrig-alkoholischen, gepufferten oder ungepufferten Zusammensetzungen (A), **dadurch gekennzeichnet, dass** es aus Folgendem besteht:
i) Herstellen, im Kalten oder bei einer Temperatur von nicht mehr als 90°C, einer pseudo-kolloidalen Lösung durch Dispersion unter Vakuum von Verdickungsmitteln, Lambda-Carrageenanen, mit einem Anteil in dem Medium der flüssigen viskosen, wässrigen oder wässrig-alkoholischen, gepufferten oder ungepufferten Zusammensetzung (A) von mehr als 2%, die Folgendes enthält:
a) Alkali- oder Erdalkaliionen
b) einen Weichmacher,
c) ein Tensid
und/oder
d) ein Sprengmittel,
ii) Halten der aus i) erhaltenen Lösung bei 25°C während ihrer Aufbewahrung,
iii) Herstellen von Filmen für die Weichkapseln bei einer bei mindestens 50°C gehaltenen Temperatur,
iv) Gelieren der Masse des Films durch Anwenden einer Komplexierungslösung (B) durch Zerstäuben und/oder durch Eintauchen entweder gleichzeitig auf die beiden Oberflächen des Films oder alternativ nach dem Ablösen des Films von seinem Träger,
wobei die Komplexierungslösungen (B) Salzlösungen von Salzen anorganischer oder organischer Säuren sowie Hydroxiden, Oxiden und Carbonaten von Calcium, Barium, Titan, Zink, Aluminium, Schwefel und Siliziumdioxiden sind;
wobei die Gelierung der flüssigen viskosen, wässrigen oder wässrig-alkoholischen Zusammensetzungen (A) unmittelbar vor Gebrauch ausgehend von den Lambda-Carrageenan-Verdickungsmitteln erhalten wird, die die einzigartige Eigenschaft aufweisen, dass sie bei Kontakt mit der Komplexierungslösung (B) sofort gelieren.

2. Herstellungsverfahren nach Anspruch 1, **dadurch gekennzeichnet, dass** die Lambda-Carrageenane in einer Konzentration von 2 bis 80 Massen-%, bezogen auf die Gesamtmasse der Zusammensetzung (A), vorliegen.

3. Herstellungsverfahren nach Anspruch 1, **dadurch gekennzeichnet, dass** der Gehalt an Tensiden von 0 bis 20 Massen-%, bezogen auf die Gesamtmasse der Zusammensetzung (A), variiert.

4. Herstellungsverfahren nach Anspruch 1, **dadurch gekennzeichnet, dass** der Anteil an Weichmacher von 0 bis 50 Massen-%, bezogen auf die Gesamtmasse der Zusammensetzung (A), variiert.

5. Herstellungsverfahren nach Anspruch 1, **dadurch gekennzeichnet, dass** die Konzentration an Feststoffen zwischen 10 und 80 Massen-%, bezogen auf die endgültige Masse der Zusammensetzung (A), beträgt.

6. Herstellungsverfahren nach Anspruch 1, **dadurch gekennzeichnet, dass** die Alkaliionen aus Natrium und Kalium ausgewählt sind.

7. Herstellungsverfahren nach Anspruch 1, **dadurch gekennzeichnet, dass** der Weichmacher zu der Klasse der Polyole des Typs Glycerin, Sorbit, Maltodextrine, Dextrose, Mannit, Xylit, Lactit, Propylenglykol, Polyoxyethylenglykol 400 bis 6000, natürlicher und halbsynthetischer Glyceride und ihrer Derivate gehört.

8. Herstellungsverfahren nach Anspruch 1, **dadurch gekennzeichnet, dass** das Tensid, das die Steuerung des Zerfalls des Films sicherstellt, zu den Klassen der ionischen, nichtionischen und amphoteren Tenside gehört.

9. Herstellungsverfahren nach Anspruch 1, **dadurch gekennzeichnet, dass** die Sprengmittel lösliche Kartoffel-, Mais-, Reis-, Maniok-, Weizenstärken sind, die chemischen oder physikalischen Umwandlungen unterworfen wurden oder nicht.

10. Herstellungsverfahren nach Anspruch 1, **dadurch gekennzeichnet, dass** die Gelierungszeit des Films in Kontakt mit der Komplexierungslösung (B) zwischen 10 Sekunden bis 10 Minuten beträgt.

11. Herstellungsverfahren nach den Ansprüchen 1 bis 10, **dadurch gekennzeichnet, dass** die Filme, die in Schritt (iv) aus den viskosen, wässrigen oder wässrig-alkoholischen, gepufferten oder ungepufferten Zusammensetzungen (A) erhalten werden, mit herkömmlichen Speiseölen oder Estern von Glycerin und Polyoxyethylenglykol, Triglyceriden, Propylenglykolestern und ihren Derivaten oder verdünnten Lösungen dieser verschiedenen Produkte geölt sind.

12. Herstellungsverfahren nach den Ansprüchen 1 bis 10, **dadurch gekennzeichnet, dass** die Einkapselung der Wirkstoffe unter Verwendung dieser Filme durch Heißverschweißen von zwei Filmen unter Druck bei einer Temperatur zwischen 50°C und 100°C erfolgt.

13. Herstellungsverfahren nach den Ansprüchen 1 bis 10, **dadurch gekennzeichnet, dass** man bei dem Verfahren den gelierten Film einem Trocknungsschritt in einem Luftstrom, dessen Temperatur zwischen -10°C und +70°C beträgt, unterzieht.

14. Herstellungsverfahren nach den Ansprüchen 1 bis 10, **dadurch gekennzeichnet, dass** man bei dem Verfahren die hergestellte Masse (Schritt iii) vor dem Gelierungsschritt (Schritt iv) einem Entgasungsschritt unter Vakuum unterzieht, um die Luft zu beseitigen, die bei der Herstellung der Filme Blasen bilden könnte.

15. Herstellungsverfahren nach den Ansprüchen 1 bis 10, **dadurch gekennzeichnet, dass** in Schritt iii) die Masse in Filmbildungssysteme entweder durch einfache Schwerkraft oder durch Druck unter Verwendung von Förderschnecken oder einer Presse überführt wird.

## Claims

1. Method for manufacturing films for manufacturing soft capsules allowing to contain aqueous and/or oily solutions from viscous aqueous or hydroalcoholic liquid compositions, buffered or not (A),
wherein it comprises:
i) producing, at cold temperatures or at a temperature not exceeding 90°C, a pseudo-colloidal solution by dispersion under vacuum of thickening agents, the lambda carrageenans, with a proportion in the medium of the viscous aqueous or hydroalcoholic liquid composition (A), buffered or not, greater than 2%, containing :
a) alkaline or alkaline-earth ions
b) a plasticizer,
c) a surfactant
and/or
d) a disintegrating agent,
ii) maintaining the resulting solution of i) at 25°C while it is stored;
iii) producing films for soft capsules at a temperature maintained at at least 50°C,
iv) gelatinizing the film mass by applying a complexing solution (B) by spraying and/or by immersion, either simultaneously on both surfaces of the film or alternately after detachment of the film from its support,
said complexing solutions (B) being saline solutions of salts of mineral or organic acids, as well as hydroxides, oxides, and carbonates of calcium, barium, titanium, zinc, aluminum, sulfur, and silicas ;
the gelatinization of viscous aqueous or hydroalcoholic liquid compositions (A) is obtained extemporaneously starting with thickening agents lambda carrageenans that exhibit the unique property of gelatinizing instantly upon contact with said complexing solution (B).

2. Manufacturing method according to claim 1, wherein the lambda carrageenans are at a concentration of 2% to 80% by weight, relative to the total weight of the composition (A) .

3. Manufacturing method according to claim 1, wherein the surfactant content varies from 0 to 20% by weight, relative to the total weight of the composition (A).

4. Manufacturing method according to claim 1, wherein the proportion of plasticizing agent varies from 0 to 50% by weight, relative to the total weight of the composition (A).

5. Manufacturing method according to claim 1, wherein the concentration of solid matter is between 10% and 80% by weight, relative to the final weight of the composition (A).

6. Manufacturing method according to claim 1, wherein the alkaline ions are selected from sodium and potassium.

7. Manufacturing method according to claim 1, wherein the plasticizing agent belongs to the class of the polyols, of the type: glycerol, sorbitol, maltodextrins, dextrose, mannitol, xylitol, lactitol, propylene glycol, polyoxyethylene glycol 400 to 6000, natural and semisynthetic glycerides, and their derivatives.

8. Manufacturing method according to claim 1, wherein the surfactant providing control of the decomposition of the film belongs to the ionic, nonionic, and amphoteric classes of surfactant.

9. Manufacturing method according to claim 1, wherein the disintegrating agents are soluble potato, corn, rice, manioc, wheat starches that have or have not undergone chemical or physical transformations.

10. Manufacturing method according to claim 1, wherein the gelatinization time of the film in contact with the complexing solution (B) is between 10 seconds and 10 minutes.

11. Manufacturing method according to claims 1 to 10, wherein the films obtained in step (iv) from said viscous aqueous or hydroalcoholic liquid compositions, buffered or not (A), are lubricated with conventional edible oils or with glycerol esters and polyoxyethylene glycol esters, triglycerides, propylene glycol esters, and their derivatives, or dilute solutions of these various products.

12. Manufacturing method according to claims 1 to 10, wherein encapsulation of the active ingredients using said films is accomplished by hot welding of the two films under pressure, at a temperature between 50°C and 100°C.

13. Manufacturing method according to claims 1 to 10, wherein it comprises subjecting the gelatinized film to a drying operation in an air stream whose temperature is between -10°C and +70°C.

14. Manufacturing method according to claims 1 to 10, wherein it comprises subjecting the produced mass (step iii), prior to the gelatinization step (step iv), to a vacuum degassing step to eliminate air, which is capable of forming bubbles during production of the films.

15. Manufacturing method according to claims 1 to 10, wherein in step iii), the mass is transferred to the film formation systems either by simple gravity or under pressure using endless screws or a press.
